# FASCICULE DE BREVET EUROPEEN

(11) **EP 1 087 747 B1**
(45) Date de publication et mention de la délivrance du brevet: **09.11.2005**
(21) Numéro de dépôt: 99925117.6
(22) Date de dépôt: 17.06.1999
(51) Int. Cl.: A61K 7/06, A61K 7/50

(54) **COMPOSITION MOUSSANTE POUR LE SOIN DES CHEVEUX**
SCHÄUMENDES HAARPFLEGEMITTEL
FOAMING COMPOSITION FOR HAIR CARE

(30) Priorité: 19.06.1998 FR 9807802
(43) Date de publication de la demande: 04.04.2001
(73) Titulaire: Galderma S.A., 6330 Cham (CH)
(72) Inventeur: PREUILH, Isabelle, F-06110 Le Cannet (FR); GUISE, Anne-Emmanuelle, F-76000 Rouen (FR); WILLCOX, Nathalie, F-06650 Le Rouret (FR)
(74) Mandataire: Casalonga, Axel
(86) Numéro de dépôt international: PCT/FR1999/001452
(87) Numéro de publication internationale: WO 1999/065456

(56) Documents cités:
- WO-A-96/01632
- WO-A-97/27835
- FR-A- 2 537 437
- FR-A- 2 718 961
- US-A- 4 722 837
- US-A- 4 835 148

## Description

La présente invention est relative à de nouvelles compositions moussantes de lavage et traitement des cheveux et/ou du cuir chevelu, contenant au moins un principe actif choisi parmi les corticostéroïdes et les rétinoïdes, un tensioactif anionique, un tensioactif amphotère et un agent propénétrant, ainsi qu'au procédé de traitement mettant en oeuvre de telles compositions.

Parmi toutes les maladies chroniques de la peau, le psoriasis est une des affections cutanées les plus communes. Cette maladie s'observe chez 1,4 à 2,9% de la population. Le cuir chevelu est l'un des sièges de prédilection du psoriasis; ce dernier provoque essentiellement des érythèmes, des desquamations, des hyperkératoses, des prurits et peut également être responsable d'une réduction de la densité capillaire. Les traitements utilisés jusqu'à présent font appel à l'acide salicylique, aux stéroïdes locaux, à l'anthraline ou au goudron de houille ou de bois. Ces traitements sont déplaisants, notamment lors de l'application de goudron, et nécessitent de longues applications, notamment lorsque des pommades capillaires sont utilisées.

En vue d'améliorer la qualité de vie du patient, sans toutefois diminuer l'effet thérapeutique du traitement, des compositions moussantes contenant des corticostéroïdes ont été mises au point. Plus particulièrement, le temps d'application de ces compositions moussantes est réduit par rapport au traitement classique.

Le brevet BE 84515 décrit une composition contenant de l'hydrocortisone dans un mélange solvant constitué de 15 à 60% d'alcool aliphatique, 15 à 60% de propylèneglycol et de 5 à 60% d'un tiers agent solubilisant pris dans le groupe constitué par le salicylate d'hydroxy-2 éthyle, le dipropylcétone et le chlorure de diméthyl cocobenzylammonium.

Le brevet EP 0 325 949 décrit une solution comprenant au moins 2,5% de corticostéroïdes, de 25 à 80% d'un tensioactif non ionique, de 0 à 70% d'éthanol, de 0 à 70% de propylèneglycol, et un agent antimicrobien.

Néanmoins, les solvants présents dans ces compositions ont tendance à s'évaporer très rapidement. De plus, la fluidité de ces compositions rend l'application difficile et il est souvent nécessaire d'appliquer ces compositions par frictionnement pour permettre une pénétration efficace des principes actifs, ce qui a pour conséquence d'irriter encore d'avantage l'épiderme, ou d'appliquer ces compositions et de les laisser agir pendant plusieurs heures, ce qui entraîne des désagréments pour le patient.

Pour éviter l'évaporation du solvant, il a été proposé dans le brevet WO 9627376 une mousse contenant un corticostéroïde, un agent casseur de mousse, un propulseur et tampon. L'agent-casseur de mousse est composé d'un alcool aliphatique, d'eau, d'un alcool gras et d'un tensioactif non ionique. Néanmoins, lorsque ces mousses sont appliquées sur des fibres kératiniques, leur aspect esthétique résultant n'est pas satisfaisant et le cuir chevelu situé sous les fibres kératiniques peut être insuffisamment traité.

La demanderesse a recherché des compositions ne présentant pas les inconvénients susmentionnés, c'est à dire une composition permettant d'améliorer la pénétration du principe actif, tout en étant d'une utilisation particulièrement aisée, présentant de bonnes propriétés cosmétiques.

La demanderesse à maintenant mis au point une composition moussante présentant les propriétés précédemment mentionnées.

Les compositions de la présente invention, tout en permettant une bonne pénétration des principes actifs, présentent également, une amélioration des performances cosmétiques des compositions pour le traitement des affections cutanées ou des cheveux, les compositions de l'invention laissant les cheveux doux, souples et ne les rendant pas gras. De plus, ces compositions sont faciles à rincer.

La demanderesse a constaté également que les compositions moussantes de la présente invention permettaient d'obtenir, de façon surprenante, une mousse présentant des caractéristiques de volume et de compacité améliorées par rapport aux compositions moussantes pour le traitement des affections cutanées ou du cuir chevelu de l'art antérieur.

Il a également été constaté que, de façon surprenante, les compositions objets de la présente invention sont stables dans le temps, en permettant d'éviter la précipitation des principes actifs, et restent donc limpides.

L'un des objets de l'invention est donc constitué par une composition moussante pour le lavage et le traitement des cheveux et/ou du cuir chevelu.

Un autre objet de l'invention est un procédé de lavage et de traitement des cheveux et/ou du cuir chevelu mettant en oeuvre une telle composition.

Un objet de l'invention est également une composition selon l'invention pour son application comme médicament.

Un objet de l'invention est aussi l'utilisation des compositions de l'invention comme médicament et pour la fabrication d'un médicament destiné à traiter les affections cutanées ou des cheveux.

D'autres objets de l'invention apparaîtront à la lecture de la description et des exemples qui suivent.

La composition moussante pour le lavage et le traitement des cheveux et/ou du cuir chevelu est essentiellement caractérisée par le fait qu'elle contient dans un milieu aqueux:
- au moins un principe actif choisi parmi les corticostéroïdes et les rétinoïdes,
- au moins un tensioactif anionique,
- au moins un tensioactif amphotère, et
- au moins un agent propénétrant.

Dans le cadre de la présente invention, les corticoïdes peuvent être choisis parmi le dipropionate d'alclométasone, l'amcinonide, le dipropionate de beclométhasone, le benzoate de béthamethasone, le dipropionate de béthaméthasone, le valérate de béthaméthasone, le budesonide, le propionate de clobétasol, préférentiellement le 17 propionate de clobétasol, le butyrate de clobétasol, le desonide, la désoximétasone, la dexaméthasone, le diacétate de diflorasone, le valerate de diflucortolone, la flurandrénolone, l'acétate de fluprednidene, le fluocortolone, le butyl de fluocortine, le fluocinonide, l'acétonide de fluocinolone, l'acétonide de fluclorolone, le pyvalate de flumétasone, le chlorhydrate de feudiline, la flumétholone, l'halcinonide, l'hydrocortisone, l'acétate d'hydrocortisone, le butyrate d'hydrocortisone, le valérate d'hydrocortisone, l'acétate de méthylprednisolone, le furoate de mométasone, la methylprednisolone, la prednisolone, l'acétonide de triamcinolone ou parmi des mélanges pharmaceutiquement acceptables de ces derniers.

Les rétinoïdes peuvent être choisis parmi l'acide t-trans rétinoïque appelé encore trétinoïne ou vitamine A acide, l'adapalène, l'isotrétinoïne, le rétinol c'est-à-dire la vitamine A et ses dérivés, tels que l'acétate, le palmitate ou le propionate de rétinol, le motrétinide, l'étrétinate, l'acitrétine, le t-trans rétinoate de zinc, des rétinoïdes de troisième génération obtenus par l'addition de groupements cycliques sur la chaîne latérale polyène, appelés encore arotinoïdes, ou des rétinoïdes de synthèse; ou parmi des mélanges pharmaceutiquement acceptables de ces derniers.

Parmi les rétinoïdes, on préfère l'adapalène, l'acide 4-[7-(1-adamantyl)-6-méthoxyétooxyméthoxy-2-naphtyl]benzoïque, l'acide 2-hydroxy-4-[3-oxo-3-(3-tert-butyl-4-méthoxyphényl)-1-propynyl]benzoïque et l'acide 4-(3,5,5,8,8,-pentaméthyl-5,6,7,8-tetrahydro-2-naphtylthio)benzoïque.

Le principe actif particulièrement préféré parmi les corticoïdes est le 17-propionate de clobetasol.

Le principe actif peut être utilisé dans des proportions de 0,001 à 5%, préférentiellement entre 0,01 à 0,3% et plus préférentiellement entre 0,05 et 0,1% en poids par rapport au poids total de la composition.

Dans le cadre de la présente invention, la nature des tensioactifs anioniques ne revêt pas de caractère véritablement critique.

Ainsi, à titre d'exemple de tensioactifs anioniques utilisables, seuls ou mélangés, on peut citer notamment les sels (en particulier sels alcalins notamment de sodium, sels d'ammonium, sels d'amines, sels d'aminoalcools ou sels de magnésium) des composés suivants: les alkylsulfates, les alkyléthersulfates, les alkylamidoéthersulfates, les alkylarylpoly-éthersulfates, les monoglycérides sulftates, les alkylsulfonates, les alkylphosphates, les alkylamidesulfonates, les alkylarylsulfonates, les α-oléfine-sulfonates, les paraffine-sulfonates, les alkylsulfosuccinates, les alkyléthersultosuccinates, les alkylamidesulfosuccinates, les alkylsulfosuccinamates, les alkylsulfoacétates, les alkylétherphosphates, les acylsarcosinates, les acylisélhionates et les N-acyltaurates, le radical alkyle ou acyle de ces différents composés comportant de préférence de 12 à 20 atomes de carbone, et le radical aryl désignant de préférence un groupement phényle ou benzyle.

Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée, les acyl-lactylales dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkyl(C₆₋C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates ou d'alkyléthersulfates ou leurs mélanges.

Plus particulièrement, on préfère utiliser le lauryléthersulfate de sodium (2 moles OE), notamment celui commercialisé sous la dénomination "Texapon N70®", le lauryléthersulfate de sodium. particulièrement celui commercialisé sous la dénomination "Sipon AOS 225 UP®", et le laurylsulfale de sodium, notamment celui commercialisé sous la dénomination "Texapon K 12®".

Ces tensioactifs anioniques peuvent être utilisés dans des proportions comprises entre 0,05 et 50%, de préférence entre 1 et 30% et plus préférentiellement entre 2 et 25% de Matière Active (M.A.) en poids par rapport au poids total de la composition.

Les tensioactifs amphotères, dont la nature ne revêt pas dans le cadre de la présente invention de caractère critique, peuvent être notamment des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate); on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoallcyl (C₁-C₆)sulfobétaïnes.

Parmi les dérivés d'amines, on peut citer les produits commercialisés sous les dénominations MIRANOL, tels que décrits dans les brevets US-2 528 378 et US-2 781 354 et de structures:

R₂ -CONHCH₂CH₂-N(R₃)(R₄)(CH₂COO⁻)

dans laquelle: R₂ désigne un radical alkyle dérivé d'un acide R₂-COOH présent dans l'huile de coprah hydrolysée, un radical heptyle, nonyle
ou undécyle, R₃ désigne un groupement bêta-hydroxyéthyle et R₄ un groupement carboxyméthyle;
et

R₅-CONCH₂CH₂-N(B)(C)

dans laquelle:
B représente -CH₂CH₂OX', C représente -(CH₂)₂ -Y', avec z = 1 ou 2,
X' désigne le groupement -CH₂CH₂-COOH ou un atome d'hydrogène
Y' désigne -COOH ou le radical -CH₂ - CHOH - SO₃H
R₅ désigne un radical alkyle d'un acide R₉-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ instaturé.

Ces composés sont classés dans le dictionnaire CTFA. 7ème édition, 1998, sous les dénominations Disodium Cocoamphodiacetate. Disodium Lauroamphodiacetate, Disodium caprylamphodiacetate, Disodium Capryloamphodiacetate, Disodium Cocoamphodipropionate, Disodium Lauroamphodipropionate, Disodium Caprylamphodipropionate, Lauroamphodipropionic acid, Cocoamphodipropionic acid.

Parmi les tensioactifs amphotères, les cocolbétaïnes sont particulièrement préférées et plus particulièrement la cocamidopropylbétaïne, notamment celle commercialisée sous la dénomination "Tegobétaïne F50®", la cocamidopropylhydroxysultaïne. notamment celle commercialisée sous la dénomination "Amonyl 675 SB®", et les cocoylbétaïnes, notamment celles commercialisées sous les dénominations "Dehyton AB 30®" et "Chimexane HC®".

Ces tensioactifs amphotères peuvent être utilisés dans des proportions comprises entre 0,01 et 30%, de préférence entre 0.5 et 20% et plus préférentiellement entre 1 et 15% en M.A. en poids par rapport au poids total de la composition.

Le ratio entre les proportions en M.A. des tensioactifs anioniques et des tensioactifs amphotères est préférentiellement compris entre 1 et 20, et plus préférentiellement entre 2 et 10.

L'agent propénétrant, qui permet de faciliter la pénétration des principes actifs est de préférence solubilisant de l'actif présent dans la composition selon l'invention. Plus particulièrement, il est choisi parmi les alcools volatiles en C₁-C₄, comme l'éthanol, l'isopropanol, parmi les alcools polyhydrique comme le propylèneglycol et parmi les éthers de glycol comme l'éthoxydiglycol.

L'agent propénétrant préféré dans le cadre de la présente invention est l'éthanol.

Les agents propénétrants peuvent être utilisés dans des concentrations comprises entre 0,1 et 25% et préférentiellement comprises entre 5 et 10% en poids par rapport au poids total de la composition.

Le ratio entre la proportion en M.A. des tensioactifs anioniques et la proportion des agents propénétrants est préférentiellement compris entre 0,1 et 10, et plus préférentiellement entre 0,5 et 5, et encore plus préférentiellement entre 1 et 2.

Les compositions objets de la présente invention pourront être épaissies et leurs propriétés cosmétiques améliorées en y ajoutant par exemple des polymères cationiques, des polymères acryliques, des dérivés cellulosiques quaternisés ou non.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660,2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire en nombre comprise entre 500 et 5.10⁶ environ, et de préférence comprise entre 10³ et 3.10⁶ environ.

Parmi les polymères cationiques, on peut citer plus particulièrement les protéines (ou hydrolysats de protéines) quaternisées et les polymères du type polyamine, polyaminoimide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment:
- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate";
- les hydrolysats de collagène portant des groupements chlorure de triméthylammonium et de triméthylstéarylammonium, vendus sous la dénomination de "Quat-Pro S" par la Société Maybroook et dénommés dans le dictionnaire CTFA"Steartrimonium Hydrolyzed Collagen";
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein";
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres:
- le "Croquat L" dont les groupements ammonium quaternaires comportent un groupement alkyle en C₁₂;
- le "Croquat M" dont les groupements ammonium quaternaires comportent des groupements alkyle en C₁₀-C₁₈;
- le "Croquat S" dont les groupements ammonium quaternaires comportent un groupement alkyle en C₁₈;
- le "Crotein Q" dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule: 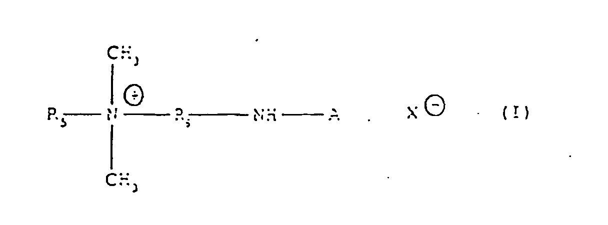 dans laquelle X^{Θ} est un anion d'un acide organique ou minéral, A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate" .

On peut encore citer les protéines végétales quaternisées telles que les protéines de blé, de maïs ou de soja: comme protéines de blé quaternisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer:
(1) Les copolymères vinylpyrrolidone-acrylate α-méthacrylate de dialkylaminoalkyle quaternisés ou non, tels que les produits vendus sous la dénomination "Gafquat" par la Société ISP, comme par exemple Gafquat 734, 755 ou HS100 ou bien le produit dénommé "Copolymère 937". Ces polymères sont décrits en détail dans les brevets français 2 077 143 et 2 393 573.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.
   Les produits commercialisés répondant à cette définition sont plus particulièrement les produits vendus sous la dénomination "Celquat SC 240", "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium .
   De tels produits sont commercialisés notamment sous les dénominations commerciales de JAGUAR C13 S, JAGUAR C 15. JAGUAR C 17 ou JAGUAR C162 par la société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bishalohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé; agent réticulant étant utilisé dans des proportions allant de 0,025 à 0,35 mole par groupement amine du polyaminoamide; ces polyaminoamides peuvent être alcoylés ou, s'ils comportent une ou plusieurs fonctions amines tertiaires, quaternisées. De tels polymères sont notamment décrits dans les brevets français 2.252.840 et 2.368.508;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8: 1 et 1,4: 1; le polyaminoamide en résultant étant amené à réagir avec l'épichlorhydrine dans un rapport molaire d' épichlorhydrine par rapport au groupement amine secondaire du polyaminoamide compris entre 0,5: 1 et 1,8: 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) les cyclohomopolymères de méthyl diallyl amine ou de diméthyl diallylammonium tels que les homopolymères comportant comme constituant principal de la chaîne des motifs répondant aux formules (VI) ou (VI'): 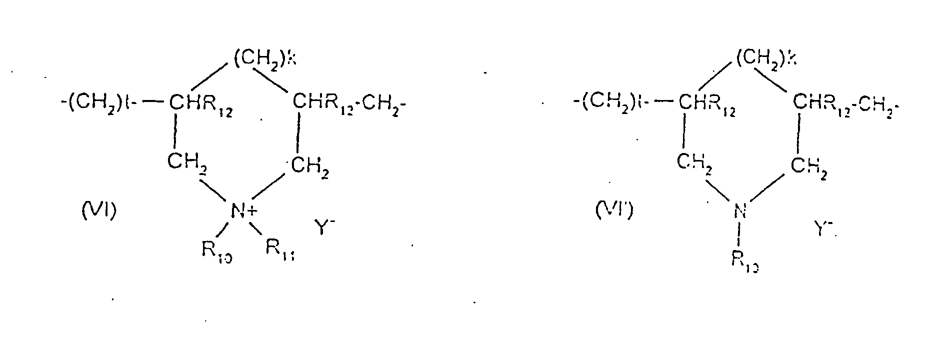 formules dans lesquelles k et t sont égaux à 0 ou 1, la somme k + t étant égale à 1; R₁₂ désigne un atome d'hydrogène ou un radical méthyle; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle; Y^{Θ} est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères défnis ci-dessus, on peut citer plus particulièrement l'homopolymère de chlorure de diméthyldiallylammonium vendu sous la dénomination "Merquat 100" par la société Merck.
(10) le polymère de diammonium quaternaire contenant des motifs récurants répondant à la formule: 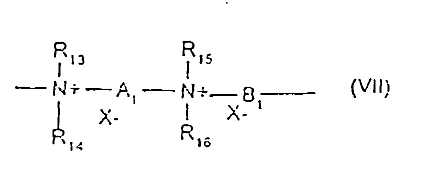 formule (VII) dans laquelle:
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire; A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et X^{Θ} désigne un anion dérivé d'un acide minéral ou organique; A₁, R₁₃ et R₁₅ peuvent former avec les deux atomes d'azote auxquels ils sont rattachés un cycle pipérazinique, en outre si A₁ désigne un radical alkylène ou hydroxyalkylène linéaire ou ramifié, saturé ou insaturé, B₁ peut également désigner un groupement (CH₂)ₙ-CO-D-OC-(CH₂)ₙ dans lequel D désigne:
      a) un reste de glycol de formule: -O-Z-O-, où Z désigne un radical hydrocarboné linéaire ou ramifié ou un groupement répondant à l'une des formules suivantes:

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen;
      b) un reste de diamine bis-secondaire tel qu'un dérivé de pipérazine;
      c) un reste de diamine bis-primaire de formule: -NH-Y-NH-, où Y désigne un radical hydrocarboné linéaire ou ramifié, ou bien le radical bivalent -CH₂-CH₂-S-S-CH₂-CH₂-;
      d) un groupement uréylène de formule: -NH-CO-NH-; De préférence, X^{Θ} est un anion tel que le chlorure ou le bromure.

      Ces polymères ont une masse moléculaire en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870. 4.001.432, 3.929.990, 3.966.904, 4.005.193, 4.025.617, 4.025.627, 4.025.653, 4.026.945 et 4.027.020.
(11) les polymères de polyammonium quaternaires constitués de motifs de formule (VIII): 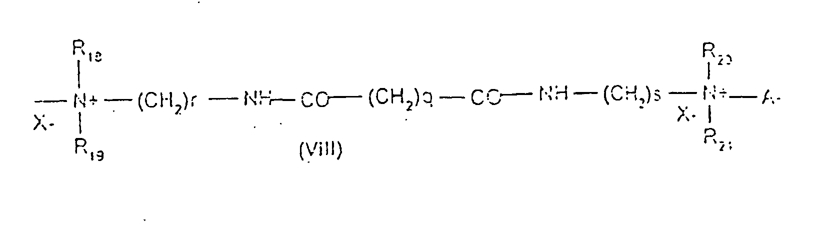 formule dans laquelle:
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
      où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6
   q est égal à 0 ou à un nombre entier compris entre 1 et 34,
   X désigne un atome d'halogène,
   A désigne un radical d'un dihalogénure ou représente de préférence -CH₂CH₂-O-CH₂-CH₂-.
      De tels composés sont notamment décrits dans la demande de brevet EP-A-122 324.
      On peut par exemple citer parmi ceux-ci, les produits "Mirapol A 15", "Mirapol AD1", "Mirapol AZ1" et "Mirapol 175" vendus par la société Miranol.
(12) les homopolymères ou copolymères dérivés des acides acrylique ou méthacrylique et comportant des motifs: 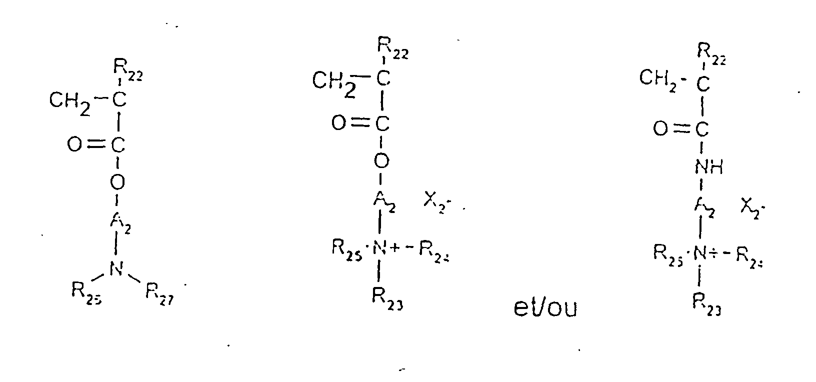 dans lesquels les groupements R₂₂ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone,
   les groupements R₂₃, R₂₄, R₂₅, identiques ou différents, désignant indépendamment un groupe alcoyle de 1 à 18 atomes de carbone ou un radical benzyle,
   les groupements R₂₆ et R₂₇ représentent un atome d'hydrogène ou un groupement alcoyle de 1 à 6 atomes de carbone,
   X₂^{Θ} désigne un anion, par exemple méthosulfate ou halogénure, tel que chlorure ou bromure.
   Le ou les comonomères utilisables dans la préparation des copolymères correspondants appartiennent à la famille des acrylamides, méthacrylamides, diacétone acrylamides, acrylamides et méthacrylamides substitués à l'azote par des alcoyles inférieurs, des esters d'alcoyles, des acides acrylique ou méthacrylique, la vinylpyrrolidone ou des esters vinyliques.
(13) Les polymères quaternaires de vinylpyrrolidone et de vinylimidazole tels que par exemple les produits commercialisés sous les dénominations Luviquat FC 905, FC 550 et FC 370 par la société B.A.S.F..
(14) Les polyamines comme le Polyquart H vendu par HENKEL, référencé sous le nom de « POLYETHYLENEGLYCOL (15) TALLOW POLYAMINE » dans le dictionnaire CTFA.
(15) Les polymères réticulés de chlorure de méthacryloyloxyéthyltriméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE SC 92» par la Société ALLIED COLLOIDS. On peut également utiliser un homopolymère réticulé du chlorure de méthacryloyloxyéthyl triméthylammonium contenant environ 50% en poids de l'homopolymère dans de l'huile minérale. Cette dispersion est commercialisée sous le nom de « SALCARE SC 95 » par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Selon l'invention, on peut utiliser plus particulièrement les polymères choisis parmi le Mirapol, le composé de formule (VII) dans laquelle R₁₃, R₁₄, R₁₅ et R₁₆ représentent le radical méthyle, A₁ représente le radical de formule -(CH₂)₃- et B₁ représente le radical de formule -(CH₂)₆- et X^{Θ} représente l'anion chlorure (nommé ultérieurement Mexomère PO) et le composé de formule (VII) dans laquelle R₁₃ et R₁₄ représentent le radical éthyle, R₁₅ et R₁₆ représentent le radical méthyle, A₁ et B₁ représentent le radical de formule -(CH₂)₃- et X^{Θ} représente l'anion bromure (nommé ultérieurement Mexomère PAK).

Parmi tous les polymères cationiques susceptibles d'être utilisés, on préfère mettre en oeuvre les composés décrits précédemment aux points (3) et (4).

Selon l'invention, le ou les polymères cationiques peuvent représenter de 0,001% à 10% en poids, de préférence de 0,005% à 5% en poids, et encore plus préférentiellement de 0,01% à 3% en poids, du poids total de la composition finale.

Le milieu aqueux peut contenir, outre de l'eau, des solvants cosmétiquement acceptables, différents de l'agent propénétrant tels que des monoalcools, des polyalcools, des éthers de glycol utilisés seuls ou en mélange.

Parmi ces solvarits, on peut plus particulièrement mentionner le polyéthylène glycol, le glycérol et le sorbitol. Les solvants sont utilisés de préférence dans des proportions de 0,5 à 10% en poids par rapport au poids total de la composition.

Le pH des compositions est de préférence compris entre 2 et 9 et en particulier entre 3 et 8. Pour le propionate de clobetasol, le pH est avantageusement compris entre 5,5 et 6,5. Il est ajusté par des agents alcalinisants ou acidifiants cosmétiquement acceptables.

Les compositions conformes à l'invention peuvent en outre contenir d'autres adjuvants utilisés dans des compositions moussantes telles que des shampooings, et notamment des céramides, telles que celles décrites dans le brevet français FR 2 673 179, des glycocéramides, des agents tensioactifs non-ioniques bien connus qui peuvent être choisis parmi les alcools, les alphadiols, les alkylphénols, les acides gras polyéthoxylés, polypropoxylés ou polyglycérolés à chaîne grasse comportant par exemple 8 à 18 atomes de carbone, le nombre de groupements d'oxyde d'éthylène et d'oxyde de propylène pouvant aller en particulier de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras; les amides gras polyéthoxylés ayant de préférence 2 à 30 moles d'oxyde d'éthylène, les amides gras polyglycérolés comportant en moyenne 1 à 5 groupements glycérol et en particulier 1,5 à 4 groupements glycérol; les amines grasses polyéthoxylées ayant de préférence 2 à 3 moles d'oxyde d'éthylène; les esters d'acides gras du sorbitan oxyéthylénés ayant de 2 à 30 moles d'oxyde d'éthylène; les esters d'acides gras du sucrose, les esters d'acides gras du polyéthyléneglycol, les alkylpolyglycosides, les dérivés de N-alkyl glycamine, les oxydes d'amines tels que les oxydes d'alkyl(C₁₀-C₁₄) amines ou les oxydes de N-acylaminopropylmorpholine. Les alkylpolyglycosides et les alcools alfadiols alkylphénols d'acides gras polyglycérolés sont plus particulièrement préférés.

Les compositions peuvent également contenir des agents épaisissants choisis notamment parmi l'alginate de sodium, la gomme arabique, les dérivés cellulosiques tels que la méthylcellulose, l'hydroxyméthylcellulose, l'hydroxyéthylcellulose, l'hydroxy-propylcellulose, l'hydroxypropylméthylcellulose, la gomme de guar ou ses dérivés, les gommes de xanthane, les scléroglucanes, les acides polyacryliques réticulés, les polyuréthanes, les copolymères à base d'acide ou d'anhydride maléique, les épaississants associatifs porteurs de chaînes grasses de type naturel comme le produit commercialisé sous la dénomination NATRASOL PLUS ou synthétiques comme les produits commercialisés sous la dénomination PEMULEN.

L'épaississant peut également être obtenu par mélange du polyéthylèneglycol et de stéarates ou de distéarates de polyéthylène glycol ou par mélange d'esters phosphoriques et d'amides.

Les compositions conformes à l'invention peuvent également contenir des colorants, des agents modificateurs de viscosité, des agents nacrants, des agents hydratants, des agents antipelliculaires, des agents antiséborrhéiques, des filtres solaires, des protéines, des vitamines, des α-hydroxyacides, des sels, des parfums, des agents conservateurs, des séquestrants, des adoucissants, des modificateurs de mousse, des détoxifiants ou leurs mélanges.

Des agents conditionneurs peuvent également être utilisés tels que les huiles naturelles hydrogénées ou non, synthétiques ou non, hydrocarbonées, cycliques ou aliphatiques, linéaires ou ramifiées (saturées ou non), les silicones volatiles ou non, organomodifiées ou non, solubles ou non, des huiles perfluorées ou fluorées, les polybutènes et polyisobutènes, des esters gras se présentant sous forme liquide, pâteuse ou solide, les esters d'alcools polyhydriques, des glycérides, les cires naturelles ou synthétiques, des gommes et résines de silicones, les sels d'ammonium quaternaire tels que par exemple le composé classé dans le dictionnaire CTFA, 7ème édition, 1998, sous la dénomination Quaternium-22 et commercialisé sous la dénomination "Ceraphyl 60", ou le mélange de ces différents agents.

Dans le cadre de la présente invention, les compositions sont plus particulièrement sous la forme de liquides, éventuellement épaissis.

Elles peuvent être utilisées en l'état ou être diluées avant utilisation.

Les compositions conformes à l'invention sont plus particulièrement utilisées comme shampooings pour le traitement des cheveux ou du cuir chevelu.

On applique, dans ce cas, préférentiellement la composition sur les cheveux mouillés ou secs puis on procède à un léger massage au cours duquel se forme une mousse, puis on rince et. éventuellement, on applique une nouvelle fois le shampooing suivi d'un nouveau rinçage à l'eau.

Un objet de l'invention est aussi une composition moussante telle que définie ci-dessus pour son application comme médicament.

La présente invention a aussi pour objet l'utilisation d'une composition telle que définie ci-dessus pour la fabrication d'un médicament destiné à traiter les affections du cuir chevelu.

Les compositions selon l'invention sont tout particulièrement indiquées dans le traitement des éczémas, des érythrodermies eczémateuses ou psoriasiques, des lésions prurigineuses, du lupus érythémateux chronique, du psoriasis et du parapsoriasis en plaques.

Ces traitements nécessitent généralement une application telle que décrite ci-dessus 2 à 3 fois par semaine.

On va maintenant donner à titre d'illustration et sans aucun caractère limitatif plusieurs exemples de compositions selon l'invention.

### EXEMPLE I

On prépare le shampooing suivant:
- Texapon N70® (Lauryléther sulfate de sodium (2 mole OE) à 70% M.A.) 17 g
- Dehyton AB 30® (Cocoylbétaïne à 32% M.A.) 6 g
- Transcutol® (ethoxydiglycol) 10 g
- Propionate de clobétasol 0,05 g
- Jaguar C162® (hydroxyméthylguar trimethylammonium) 0,5 g
- Acide lactique qs pH6
- Eau déminéralisée qs 100 g

### EXEMPLE II

On prépare le shampooing suivant:
- Dehyton AB 30 (cocoylbétaïne à 32% M.A.) 6 g
- Jaguar C162®(hydroxyméthylguar trimethylammonium) 0,5 g
- Sipon AOS 225 UP® (lauryléther sulfate de sodium à 28% M.A.) 43 g
- Ethanol (95/96%) 10 g
- Propionate de clobétasol 0.05 g
- Chlorure de benzalkonium 0,005 g
- Acide lactique qs pH6
- Eau déminéralisée qs 100 g

### EXEMPLE III

On prépare le shampooing suivant:
- Texapon N70® (Lauryléther sulfate de sodium (2 mole OE) à 70% M.A.) 17 g
- Dehyton AB 30® (cocoylbetaïne à 32% M.A.) 6 g
- Transcutol® (ethoxydiglycol) 10 g
- Adapalène 0,05 g
- Jaguar C162® (hydroxymethylguar trimethylammonium) 0,5 g
- Acide lactique qs pH6
- eau déminéralisée qs 100 g

### EXEMPLE I V

On prépare le shampooing suivant:
- Sipon AOS 225 UP® (lauryléther sulfate de sodium à 28% M.A.) 43 g
- Dehyton AB 30® (cocoylbetaïne à 31% M.A.) 6 g
- Ethanol (95/96%) 10 g
- Chlorure de benzalkonium 0.01 g
- Jaguar C 162® (hydroxymethylguar trimethylammonium) 0,5 g
- Propionate de clobétasol 0,05 g
- Acide lactique qs pH 6
- eau déminéralisée qs 100 g

### EXEMPLE V

On prépare le shampooing suivant:
- Celquat SC 240® (Polyquaternium 10) 2 g
- Texapon N70® (Lauryléther sul fate de sodium (2 moles OE) à 70% M.A.) 17 g
- Dehyton AB 30® (Cocoylbétaïne à 32% M.A.) 6 g
- Rewoquat B50® (Chlorure de benzalkonium 50%) 0,01 g
- Acide citrique, 1H₂O 0,24 g
- Citrate de sodium, 2H₂O 2,6 g
- Ethanol (95/96%) 10 g
- Propionate de clobétasol 0.05 g
- Eau déminéralisée qs 100 g

### EXEMPLE VI

On prépare le shampooing suivant:
- Celquat SC 240® (Polyquaternium 10) 2 g
- Texapon N70® (Lauryléther sulfate de sodium (2 moles OE) à 70% M.A.) 17 g
- Dehyton AB 30® (Cocoylbétaïne à 32% M.A.) 6 g
- Rewoquat B50® (Chlorure de benzalkonium 50%) 0,01 g
- Ethanol (95/96%) 10 g
- Propionate de clobétasol 0,05 g
- Eau déminéralisée qsp 100 g

### EXEMPLE VII

On prépare le shampooing suivant:
- Acide citrique, 1H₂O 0,24 g
- Citrate de sodium, 2H₂O 2,6 g
- Parahydroxybenzoate de méthyl 0,1 g
- Celquat SC 240® (polyquaternium 10) 2 g
- Texapon N70® (Lauryléther sulfate de sodium (2 moles OE) à 70% M.A.) 17 g
- Dehyton AB 30® (Cocoylbétaïne à 32% M.A.) 6 g
- Propionate de clobétasol 0,05 g
- Ethanol (95/96%) 10 g
- Eau purifiée qsp 100 g

### EXEMPLE VIII

On prépare le shampooing suivant:
- Acide citrique 0,24 g
- Citrate de sodium 2.6 g
- Celquat SC 240® (Polyquaternium 10) 2 g
- Texapon N70®(Lauryléther sulfate de sodium (2 moles OE) à 70% M.A.) 17 g
- Dehyton AB 30® (Cocoylhétaïne à 32% M.A.) 6 g
- Propionate de Clobetasol 0,05 g
- Ethanol (95/96%) 10 g
- Eau purifiée qsp 100 g

### EXEMPLE IX

On prépare le shampooing suivant:
- Jaquar C 162®(hydroxymethylguar trimethylammonium) 0.5 g
- Chimexane HC (Cocoylbétaïne à 32% M.A.) 6,0 g
- Sipon AOS 225 UP® (lauryléther sulfate de sodium à 28% M.A.) 43,0 g
- Ethanol (95/96%) 10,0 g
- Propionate de clobétasol 0,05 g
- Rewoquat B50 (Chlorure de benzalkonium 50%) 0,0 1 g
- Eau purifiée qsp 100 g

### EXEMPLE X

On prépare le shampooing suivant:
- Jaquar C162® (hydroxyméthyl guar trimethylammonium) 0,5 g
- Texapon N70® (Lauryléther sulfate de sodium (2 moles OE) à 70% M.A.) 17 g
- Dehyton AB 30® (Cocoylbétaïne à 32% M.A.) 6 g
- Rewoquat B50® (Chlorure de benzalkonium 50%) 0,01 g
- Ethanol (95/96%) 10 g
- Adapalène 0,05 g
- Eau déminéralisée qsp 100 g

### EXEMPLEXI

On prépare le shampooing suivant:
- Jaquar C162® (hydroxyméthyl guar trimethylammonium) 0,5 g
- Texapon N70® (Lauryléther sulfate de sodium (2 moles OE) à 70% M.A.) 17 g
- Dehyton AB 30® (Cocoylbétaïne à 32% M.A.) 6 g
- Rewoquat B50® (Chlorure de benzalkonium 50%) 0,01 g
- Ethanol (95/96%) 10 g
- Acide 4-[7-(1-adamantyl)-6-méthoxy éthoxy méthoxy
- 2-napthyl]benzoïque 0,05 g
- Eau déminéralisée qsp 100 g

### EXEMPLE XII

On prépare le shampooing suivant:
- Jaquar C162® (hydroxyméthyl guar trimethylammonium) 0,5 g
- Texapon N70® (Lauryléther sulfate de sodium (2 moles OE) à 70% M.A.) 17 g
- Dehyton AB 30® (Cocoylbétaïne à 32% M.A.) 6 g
- Rewoquat B50® (Chlorure de benzalkonium 50%) 0,01 g
- Ethanol (9S/96%) 10 g
- Acide 2-hydroxy-4-[3-oxo-3-(3-tert-butyl-4-méthoxyphényl)-1-propynyl] benzoïque 0,05 g
- Eau déminéralisée qsp 100 g

### EXEMPLE XIII

On prépare le shampooing suivant:
- Jaquar C162® (hydroxyméthyl guar trimethylammonium) 0,5 g
- Texapon N70® (Lauryléther sulfate de sodium (2 moles OE) à 70% M.A.) 17 g
- Dehyton AB 30® (Cocoylbétaïne à 32% M.A.) 6 g
- Rewoquat B50® (Chlorure de benzalkonium 50%) 0,01 g
- Ethanol (95/96%) 10 g
- Acide 4-(3,5,5,8,8-pentaméthyl-5,6,7,8 tetrahydro-2-naphtylthio)benzoïque 0,05 g
- Eau déminéralisée qsp 100 g

### EXEMPLE XIV

On prépare le shampooing suivant:
- Texapon N70® (Lauryléther sulfate de sodium (2 moles OE) à 70% M.A.) 17 g
- Dehyton AB 30® (Cocoylbétaïne à 32% M.A.) 6 g
- Methocel E4M(hydroxy propyl methyl cellulose) 1 g
- Acide citrique, 1H₂O 0,24 g
- Citrate de sodium, 2H₂O 2,6 g
- Ceraphyl 60 (Quaternium 22) 0.5 g
- Propionate de clobétasol 0,05 g
- Ethanol (95/96%) 10 g
- Eau déminéralisée qs 100 g

Les compositions des exemples I à XIV ci-dessus sont stables au stockage et présentent un effet moussant satisfaisant.

Une étude clinique, dans laquelle une composition selon l'exemple IX a été utilisée comme shampooing une fois par jour pendant deux semaines, le shampooing étant appliqué sur cheveux mouillés et laissé en contact pendant 10 minutes, pour être ensuite rincé, a permis de constater chez des patients atteints de psoriasis, une réduction au niveau du cuir chevelu des érythèmes de 37%, de la desquamation de 47%, des hyperkératoses de 50% et des prurits de 57%.

## Revendications

1. Composition moussante pour le lavage et le traitement des cheveux et/ou du cuir chevelu **caractérisée par le fait qu'**elle contient dans un milieu aqueux:
- au moins un principe actif choisi parmi les corticoïdes et les rétinoïdes,
- au moins un tensioactif anionique,
- au moins un tensioactif amphotère, et
- au moins un agent propénétrant.

2. Composition selon la revendication 1, **caractérisée par le fait que** les corticoïdes sont choisis parmi le dipropionate d'alclométasone, l'amcinonide, le dipropionate de beclaméthasone, le benzoate de béthamethasone, le dipropionate de béthaméthasone, le valérate de béthaméthasone, le budesonide, le propionate de clobétasol, préférentiellement le 17 propionate de clobétasol, le butyrate de clobétasol, le desonide, la désoximétasone, la dexaméthasone, le diacétate de diflorasone, le valerate de diflucortolone, la flurandrénolone, l'acétate de fluprednidene, le fluocortolone, le butyl de fluocortine, le fluocinonide, l'acétonide de fluocinolone, l'acétonide de fluclorolone, le pyvalate de flumétasone, le chlorhydrate de feudiline, la flumétholone, l'halcinonide, l'hydrocortisone, l'acétate d'hydrocortisone, le butyrate d'hydrocortisone, le valérate d'hydrocortisone, l'acétate de méthylprednisolone, le furoate de mométasone, la methylprednisolone, la prednisolone, l'acétonide de triamcinolone ou parmi des mélanges pharmaceutiquement acceptables de ces derniers.

3. Composition selon la revendication 1 ou 2, **caractérisée par le fait que** les rétinoïdes sont choisis parmi l'acide t-trans rétinoïque, l'adapalène, l'isotrétinoïne, le rétinol et ses dérivés, tels que l'acétate, le palmitate ou le propionate de rétinol, le motrétinide, l'étrétinate, l'acitrétine, le t-trans rétinoate de zinc, les arotinoïdes, les rétinoïdes de synthèse ou parmi des mélanges pharmaceutiquement acceptables de ces derniers.

4. Composition selon la revendication 1 ou 2, **caractérisée par le fait que** le principe actif est choisi parmi le 17-propionate de clobétasol, l'adapalène, l'acide 4-[7-(1-adamantyl)-6-méthoxyéthoxyméthoxy-2-naphtyl]benzoïqué, l'acide 2-hydroxy-4-[3-oxo-3-(3-tert-butyl-4-méthoxyphényl)-1-propynyl]benzoïque et l'acide 4-(3,5,5,8,8,-pentamethyl-5,6,7,8-tetrahydro-2-naphtylthio)benzoïque.

5. Composition selon l'une quelconque des revendications 1 à 3, **caractérisée par le fait que** les tensioactifs anioniques sont choisis parmi les sels des composés suivants: les alkylsulfates, les alkyléthersulfates, les alkylamidoéthersulfates, les alkylarylpoly-éthersulfates, les monoglycérides sulftates, les alkylsulfonates, les alkylphosphates, les alkylamidesulfonates, les alkylarylsulfonates, les α-oléfine-sulfonates, les paraffine-sulfonates, les alkylsulfosuccinates, les alkyléthersulfosuccinates, les alkylamidesulfosuccinates, les alkylsulfosuccinamates, les alkylsulfoacétates, les alkylétherphosphates, les acylsarcosinates, les acyliséthionates et les N-acyltaurates, et parmi les sels des acides oléique, ricinoléique, palmitique, stéarique, les acides d'huile de coprah ou d'huile de coprah hydrogénée, les acyl-lactylates; et parmi les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels.

6. Composition selon l'une quelconque des revendications 1 à 5, **caractérisée par le fait que** les tensioactifs anioniques sont choisis parmi les sels d'alkylsulfates et d'alkyléthersulfates.

7. Composition selon la revendication 6, **caractérisée par le fait que** les tensioactifs anioniques sont choisis parmi le laurylethersulfate de sodium et le laurylsulfate de sodium.

8. Composition selon l'une quelconque des revendications 1 à 7, **caractérisée par le fait que** les tensioactifs amphotères sont choisis parmi les dérivés d'amines secondaires ou tertiaires aliphatiques, les alkyl (C₈-C₂₀) bétaïnes, les sulfobétaïnes, les alkyl (C₈-C₂₀) amidoalkyl (C₁-C₆) bétaïnes et les alkyl (C₈-C₂₀) amidoalkyl (C₁-C₆)sulfobétaïnes.

9. Composition selon l'une quelconque des revendications 1 à 8, **caractérisée par le fait que** les tensioactifs amphotères sont choisis parmi les cocobétaïnes.

10. Composition selon la revendication 9, **caractérisée par le fait que** les tensioactifs amphotères sont choisis parmi les cocamidopropylbétaïnes, les cocamidopropylhydroxysultaïne, les cocoylbétaïnes.

11. Composition selon l'une quelconque des revendications 1 à 10, **caractérisée par le fait que** l'agent propénétrant est choisi parmi les alcools volatiles en C₁-C₄, et parmi les alcools polyhydriques.

12. Composition selon l'une quelconque des revendications 1 à 11, **caractérisée par le fait que** l'agent propénétrant est choisi parmi l'éthanol, l'isopropanol, le propylèneglycol et l'éthoxydiglycol.

13. Composition selon l'une quelconque des revendications 1 à 12, **caractérisée par le fait que** le principe actif est utilisé dans des proportions de 0,001 à 5%, préférentiellement entre 0,01 à 0,3% et plus préférentiellement entre 0,05 et 0,1% en poids par rapport au poids total de la composition.

14. Composition selon l'une quelconque des revendications 1 à 13, **caractérisée par le fait que** le tensioactif anionique est utilisé dans des proportions de 0,05 et 50%, de préférence entre 1 et 30% et plus préférentiellement entre 2 et 25% de M.A. en poids par rapport au poids total de la composition.

15. Composition selon l'une quelconque des revendications 1 à 14, **caractérisée par le fait que** le tensioactif amphotère est utilisé dans des proportions de 0,01 et 30%, de préférence entre 0,5 et 20% et plus préférentiellement entre 1 et 15% en M.A. en poids par rapport au poids total de la composition.

16. Composition selon l'une quelconque des revendications 1 à 15, **caractérisée par le fait que** l'agent propénétrant est utilisé dans des concentrations comprises entre 0,1 et 25% et préférentiellement comprises entre 5 et 10% en poids par rapport au poids total de la composition.

17. Composition selon l'une quelconque des revendications 1 à 16, **caractérisée en ce que** le ratio entre les proportions en M.A. des tensioactifs anioniques et amphotères est compris entre 1 et 20 et, préférentiellement entre 2 et 10.

18. Composition selon l'une quelconque des revendications 1 à 17, **caractérisée en ce que** le ratio entre la proportion en M.A. des tensioactifs anioniques et la proportion des agents propénétrants est compris entre 0,1 et 10, préférentiellement entre 0,5 et 5.

19. Composition selon l'une quelconque des revendications 1 à 18, **caractérisée par le fait que** la composition contient en outre des polymères cationiques choisis parmi les protéines quaternisées, les polymères de type polyamine, polyaminoamide, polyammonium quaternaire, les polyalkylèneimines, des condensats de polyamines et d'épichlorhydrine des polyuréylènes quaternaires et les dérivés de la chitine.

20. Composition selon la revendication 19, **caractérisée par le fait que** les polymères cationiques sont choisis parmi les dérivés d'éther de celluloses quaternaires, les cyclopolymères, et les polysaccharides cationiques.

21. Composition selon la revendication 19 ou 20, **caractérisée par le fait que** le polymère cationique représente 0,001 % à 10 % en poids, de préférence de 0,005 % à 5 % en poids, et encore plus préférentiellement de 0,01 % à 3 % en poids, par rapport au poids total de la composition finale.

22. Composition selon l'une quelconque des revendications 1 à 21, **caractérisée par le fait que** la pH des compositions est compris entre 2 et 9, préférentiellement entre 3 et 8 et plus préférentiellement entre 5,5 et 6,5.

23. Composition selon l'une quelconque des revendications 1 à 22, **caractérisée par le fait que** la composition contient également des agents tensio-actifs non-ioniques et/ou des céramides et/ou des glycocéramides.

24. Composition selon l'une quelconque des revendications 1 à 23, **caractérisée par le fait que** la composition contient des agents épaississants.

25. Composition selon l'une quelconque des revendications 1 à 24, **caractérisée par le fait que** la composition contient également des colorants, des agents modificateurs de viscosité, des agents nacrants, des agents hydratants, des agents antipelliculaires, des agents antiséborrhéiques, des filtres solaires, des protéines, des vitamines, des α-hydroxyacides, des sels, des parfums, des agents conservateurs, des séquestrants, des adoucissants, des modificateurs de mousse, des détoxifiants ou leurs mélanges.

26. Composition selon l'une quelconque des revendications 1 à 25, **caractérisée par le fait que** la composition contient également des agents conditionneurs tels que les huiles naturelles hydrogénées ou non, synthétiques ou non, hydrocarbonées, cycliques ou aliphatiques, linéaires ou ramifiées (saturées ou non), les silicones volatiles ou non, organomodifiées ou non, solubles ou non, des huiles perfluorées ou fluorées, les polybutènes et polyisobutènes, des esters gras se présentant sous forme liquide, pâteuse ou solide, les esters d'alcools polyhydriques, des glycérides, les cires naturelles ou synthétiques, des gommes et résines de silicones, des sels quaternaires ou le mélange de ces différents agents.

27. Composition selon la revendication 26, **caractérisée par le fait que** la composition contient également du quaternium-22.

28. Composition selon l'une quelconque des revendications 1 à 27, **caractérisée par le fait qu'**elle se présente sous forme de liquides, éventuellement épaissis.

29. Composition selon l'une quelconque des revendications 1 à 28, pour son application comme médicament.

30. Utilisation de la composition telle que définie dans l'une quelconque des revendications 1 à 28, pour la fabrication d'un médicament destiné à traiter les affections du cuir chevelu.

## Claims

1. Foaming composition for washing and treating the hair and/or the scalp, **characterized in that** it comprises, in an aqueous medium:
- at least one active principle chosen from corticoids and retinoids,
- at least one anionic surfactant,
- at least one amphoteric surfactant, and
- at least one propenetrating agent.

2. Composition according to Claim 1, **characterized in that** the corticoids are chosen from alclometasone dipropionate, amcinonide, beclomethasone dipropionate, betamethasone benzoate, betamethasone dipropionate, betamethasone valerate, budesonide, clobetasol propionate, preferably clobetasol 17-propionate, clobetasol butyrate, desonide, desoximetasone, dexamethasone, diflorasone diacetate, diflucortolone valerate, flurandrenolone, fluprednidene acetate, fluocortolone, fluocortin butyl, fluocinonide, fluocinolone acetonide, fluclorolone acetonide, flumetasone pivalate, feudiline hydrochloride, flumetholon, halcinonide, hydrocortisone, hydrocortisone acetate, hydrocortisone butyrate, hydrocortisone valerate, methylprednisolone acetate, mometasone furoate, methylprednisolone, prednisolone or triamcinolone acetonide or from pharmaceutically acceptable mixtures of the latter.

3. Composition according to Claim 1 or 2, **characterized in that** the retinoids are chosen from all-trans-retinoic acid, adapalene, isotretinoin, retinol and its derivatives, such as retinol acetate, palmitate or propionate, motretinide, etretinate, acitretin, zinc all-trans-retinoate, arotinoids or synthetic retinoids or from pharmaceutically acceptable mixtures of the latter.

4. Composition according to Claim 1 or 2, **characterized in that** the active principle is chosen from clobetasol 17-propionate, adapalene, 4-[7-(1-adamantyl)-6-methoxyethoxymethoxy-2-naphthyl]benzoic acid, 2-hydroxy-4-[3-oxo-3-(3-tert-butyl-4-methoxyphenyl)-1-propynyl]benzoic acid and 4-(3,5,5,8,8-pentamethyl-5,6,7,8-tetrahydro-2-naphthylthio)benzoic acid.

5. Composition according to any one of Claims 1 to 3, **characterized in that** the anionic surfactants are chosen from the salts of the following compounds: alkyl sulphates, alkyl ether sulphates, alkylamido ether sulphates, alkylaryl polyether sulphates, monoglyceride sulphates, alkyl sulphonates, alkyl phosphates, alkylamide sulphonates, alkylaryl sulphonates, α-olefin sulphonates, paraffin sulphonates, alkyl sulphosuccinates, alkyl ether sulphosuccinates, alkylamide sulphosuccinates, alkylsulphosuccinamates, alkyl sulphoacetates, alkyl ether phosphates, acylsarcosinates, acylisethionates and N-acyltaurates, and from the salts of oleic, ricinoleic, palmitic and stearic acids, the acids of coconut oil or of hydrogenated coconut oil, or acyllactylates; and from alkyl D-galactosideuronic acids and their salts, as well as polyoxyalkylenated (C₆-C₂₄)alkyl ether carboxylic acids, polyoxyalkylenated (C₆-C₂₄)alkylaryl ether carboxylic acids, polyoxyalkylenated (C₆-C₂₄)alkylamido ether carboxylic acids and their salts.

6. Composition according to any one of Claims 1 to 5, **characterized in that** the anionic surfactants are chosen from alkyl sulphate and alkyl ether sulphate salts.

7. Composition according to Claim 6, **characterized in that** the anionic surfactants are chosen from sodium lauryl ether sulphate and sodium lauryl sulphate.

8. Composition according to any one of Claims 1 to 7, **characterized in that** the amphoteric surfactants are chosen from derivatives of aliphatic secondary or tertiary amines, (C₈-C₂₀)alkyl betaines, sulphobetaines, (C₈-C₂₀)alkyl amido(C₁-C₆)alkyl betaines or (C₈-C₂₀)alkyl amido(C₁-C₆)alkyl sulphobetaines.

9. Composition according to any one of Claims 1 to 8, **characterized in that** the amphoteric surfactants are chosen from cocobetaines.

10. Composition according to Claim 9, **characterized in that** the amphoteric surfactants are chosen from cocamidopropyl betaines, cocamidopropyl hydroxysultaine or cocoyl betaines.

11. Composition according to any one of Claims 1 to 10, **characterized in that** the propenetrating agent is chosen from volatile C₁-C₄ alcohols and from polyhydric alcohols.

12. Composition according to any one of Claims 1 to 11, **characterized in that** the propenetrating agent is chosen from ethanol, isopropanol, propylene glycol and ethoxydiglycol.

13. Composition according to any one of Claims 1 to 12, **characterized in that** the active principle is used in proportions of 0.001 to 5%, preferably between 0.01 and 0.3% and more preferably between 0.05 and 0.1% by weight with respect to the total weight of the composition.

14. Composition according to any one of Claims 1 to 13, **characterized in that** the anionic surfactant is used in proportions of between 0.05 and 50%, preferably between 1 and 30% and more preferably between 2 and 25% of A.M. by weight with respect to the total weight of the composition.

15. Composition according to any one of Claims 1 to 14, **characterized in that** the amphoteric surfactant is used in proportions of between 0.01 and 30%, preferably between 0.5 and 20% and more preferably between 1 and 15% as A.M. by weight with respect to the total weight of the composition.

16. Composition according to any one of Claims 1 to 15, **characterized in that** the propenetrating agent is used in concentrations of between 0.1 and 25% and preferably of between 5 and 10% by weight with respect to the total weight of the composition.

17. Composition according to any one of Claims 1 to 16, **characterized in that** the ratio of the proportion as A.M. of the anionic surfactants to the proportion as A.M. of the amphoteric surfactants is between 1 and.20 and preferably between 2 and 10.

18. Composition according to any one of Claims 1 to 17, **characterized in that** the ratio of the proportion as A.M. of the anionic surfactants to the proportion of the propenetrating agents is between 0.1 and 10, preferably between 0.5 and 5.

19. Composition according to any one of Claims 1 to 18, **characterized in that** the composition additionally comprises cationic polymers chosen from quaternized proteins, polymers of polyamine, polyaminoamide or poly(quaternary ammonium) type, polyalkyleneimines, condensates of polyamines and of epichlorohydrin, quaternary polyureylenes and chitin derivatives.

20. Composition according to Claim 19, **characterized in that** the cationic polymers are chosen from quaternary cellulose ether derivatives, cyclopolymers and cationic polysaccharides.

21. Composition according to Claim 19 or 20, **characterized in that** the cationic polymer represents 0.001% to 10% by weight, preferably from 0.005% to 5% by weight and more preferably still from 0.01% to 3% by weight with respect to the total weight of the final composition.

22. Composition according to any one of Claims 1 to 21, **characterized in that** the pH of the compositions is between 2 and 9, preferably between 3 and 8 and more preferably between 5.5 and 6.5.

23. Composition according to any one of Claims 1 to 22, **characterized in that** the composition also comprises nonionic surfactants and/or ceramides and/or glycoceramides.

24. Composition according to any one of Claims 1 to 23, **characterized in that** the composition comprises thickening agents.

25. Composition according to any one of Claims 1 to 24, **characterized in that** the composition also comprises colourants, viscosity-modifying agents, pearlescent agents, moisturizing agents, antidandruff agents, antiseborrheic agents, sunscreens, proteins, vitamins, α-hydroxy acids, salts, fragrances, preservatives, sequestering agents, softeners, foam modifiers, detoxifying agents or their mixtures.

26. Composition according to any one of Claims 1 to 25, **characterized in that** the composition also comprises conditioning agents, such as hydrogenated or nonhydrogenated and synthetic or nonsynthetic natural hydrocarbonaceous oils which are cyclic or aliphatic and linear or branched (saturated or unsaturated), volatile or nonvolatile, organomodified or nonorganomodified and soluble or insoluble silicones, perfluorinated or fluorinated oils, polybutenes and polyisobutenes, fatty esters which are provided in a liquid, pasty or solid form, esters of polyhydric alcohols, glycerides, natural or synthetic waxes, silicone gums and resins, quaternary ammonium salts or the mixture of these various agents.

27. Composition according to Claim 26, **characterized in that** the composition also comprises quaternium-22.

28. Composition according to any one of Claims 1 to 27, **characterized in that** it is provided in the form of liquids, optionally thickened liquids.

29. Composition according to any one of Claims 1 to 28 for its application as medicament.

30. Use of the composition as defined in any one of Claims 1 to 28 in the manufacture of a medicament intended for the treatment of ailments of the scalp.

## Patentansprüche

1. Schäumende Zusammensetzung für die Reinigung und die Behandlung der Haare und/oder der Kopfhaut, **dadurch gekennzeichnet, dass** sie in einem wässrigen Medium enthält:
- mindestens einen Wirkstoff, der unter den Corticoiden und Retinoiden ausgewählt ist,
- mindestens einen anionischen grenzflächenaktiven Stoff,
- mindestens einen amphoteren grenzflächenaktiven Stoff und
- mindestens einen die Penetration fördernden Wirkstoff.

2. Zusammensetzung nach Anspruch 1, **dadurch gekennzeichnet, dass** die Corticoide unter Alclometasondipropionat, Amcinonid, Beclamethasondipropionat, Bethamethasonbenzoat, Bethamethasondipropionat, Bethamethasonvalerat, Budesonid, Clobetasolpropionat, vorzugsweise Clobetasol-17-propionat, Clobetasolbutyrat, Desonid, Desoximetason, Dexamethason, Diflorasondiacetat, Diflucortolonvalerat, Flurandrenolon, Fluprednidenacetat, Fluocortolon, Fluocortinbutyl, Fluocinonid, Fluocinolonacetonid, Fluclorolonacetonid, Flumetasonpivalat, Feudilin-Hydrochlorid, Flumetholon, Halcinonid, Hydrocortison, Hydrocortisonacetat, Hydrocortisonbutyrat, Hydrocortisonvalerat, Methylprednisolonacetat, Mometasonfuroat, Methylprednisolon, Prednisolon, Triamcinolonacetonid oder unter den pharmazeutisch akzeptablen Gemischen dieser Wirkstoffe ausgewählt sind.

3. Zusammensetzung nach Anspruch 1 oder 2, **dadurch gekennzeichnet, dass** die Retinoide unter all-trans-Retinsäure, Adapalen, Isotretinoin, Retinol und seinen Derivaten, wie dem Acetat, Palmitat oder Propionat von Retinol, Motretinid, Etretinat, Acitretin, Zink-all-trans-retinoat, Arotinoiden, synthetischen Retinoiden oder unter den pharmazeutisch akzeptablen Gemischen dieser Wirkstoffe ausgewählt sind.

4. Zusammensetzung nach Anspruch 1 oder 2, **dadurch gekennzeichnet, dass** der Wirkstoff unter Clobetasol-17-propionat, Adapalen, 4-[7-(1-Adamantyl)-6-methoxyethoxymethoxy-2-naphthyl]benzoesäure, 2-Hydroxy-4-[3-oxo-3-(3-t-butyl-4-methoxyphenyl)-1-propinyl]benzoesäure und 4-(3,5,5,8,8-Pentamethyl-5,6,7,8-tetrahydro-2-naphthylthio)benzoesäure ausgewählt ist.

5. Zusammensetzung nach einem der Ansprüche 1 bis 3, **dadurch gekennzeichnet, dass** die anionischen grenzflächenaktiven Stoffe unter den Salzen der folgenden Verbindungen ausgewählt sind: Alkylsulfate, Alkylethersulfate, Alkylamidoethersulfate, Alkylarylpolyethersulfate, Monoglyceridsulfate, Alkylsulfonate, Alkylphosphate, Alkylamidsulfonate, Alkylarylsulfonate, α-Olefinsulfonate, Paraffinsulfonate, Alkylsulfosuccinate, Alkylethersulfosuccinate, Alkylamidsulfosuccinate, Alkylsulfosuccinamate, Alkylsulfoacetate, alkyletherphosphate, Acylsarcosinate, Acylisethionate und N-Acyltaurate; und unter den Salzen von Ölsäure, Ricinolsäure, Palmitinsäure, Stearinsäure, Säuren von Kopraöl oder hydriertem Kopraöl, Acyllactylaten; und unter den Alkyl-D-galactosiduronsäuren und deren Salzen sowie polyalkoxylierten Alkyl(C₆₋₂₄)ethercarbonsäuren, polyalkoxylierten Alkyl-(C₆₋₂₄)arylethercarbonsäuren, polyalkoxylierten Alkyl(C₆₋₂₄)amido-ethercarbonsäuren und deren Salzen.

6. Zusammensetzung nach einem der Ansprüche 1 bis 5, **dadurch gekennzeichnet, dass** die anionischen grenzflächenaktiven Stoffe unter den Salzen von Alkylsulfaten und Alkylethersulfaten ausgewählt sind.

7. Zusammensetzung nach Anspruch 6, **dadurch gekennzeichnet, dass** die anionischen grenzflächenaktiven Stoffe unter Natriumlaurylethersulfat und Natriumlaurylsulfat ausgewählt sind.

8. Zusammensetzung nach einem der Ansprüche 1 bis 7, **dadurch gekennzeichnet, dass** die amphoteren grenzflächenaktiven Stoffe unter den aliphatischen, sekundären oder tertiären Aminderivaten, Alkyl(C₈₋₂₀)betainen, Sulfobetainen, Alkyl(C₈₋₂₀)amidoalkyl-(C₁₋₆)betainen und Alkyl(C₈₋₂₀)amidoalkyl(C₁₋₆)sulfobetainen ausgewählt sind.

9. Zusammensetzung nach einem der Ansprüche 1 bis 8, **dadurch gekennzeichnet, dass** die amphoteren grenzflächenaktiven Stoffe unter den Cocobetainen ausgewählt sind.

10. Zusammensetzung nach Anspruch 9, **dadurch gekennzeichnet, dass** die amphoteren grenzflächenaktiven Stoffe unter den Cocamidopropylbetainen, Cocamidopropylhydroxysultainen und Cocoylbetainen ausgewählt sind.

11. Zusammensetzung nach einem der Ansprüche 1 bis 10, **dadurch gekennzeichnet, dass** der die Penetration fördernde Wirkstoff unter den flüchtigen C₁₋₄-Alkoholen und mehrwertigen Alkoholen ausgewählt ist.

12. Zusammensetzung nach einem der Ansprüche 1 bis 11, **dadurch gekennzeichnet, dass** der die Penetration fördernde Wirkstoff unter Ethanol, Isopropanol, Propylenglykol und Ethoxydiglykol ausgewählt ist.

13. Zusammensetzung nach einem der Ansprüche 1 bis 12, **dadurch gekennzeichnet, dass** der Wirkstoff in Mengenanteilen von 0,001 bis 5 Gew.-%, vorzugsweise 0,01 bis 0,3 Gew.-% und insbesondere 0,05 bis 0,1 Gew.-%, bezogen auf das Gesamtgewicht der Zusammensetzung, verwendet wird.

14. Zusammensetzung nach einem der Ansprüche 1 bis 13, **dadurch gekennzeichnet, dass** der anionische grenzflächenaktive Stoff in Mengenanteilen von 0,05 bis 50 Gew.-%, vorzugsweise 1 bis 30 Gew.-% und noch bevorzugter 2 bis 25 Gew.-% wirksame Substanz, bezogen auf das Gesamtgewicht der Zusammensetzung, verwendet wird.

15. Zusammensetzung nach einem der Ansprüche 1 bis 14, **dadurch gekennzeichnet, dass** der amphotere grenzflächenaktive Stoff in Mengenanteilen von 0,01 bis 30 Gew.-%, vorzugsweise 0,5 bis 20 Gew.-% und noch bevorzugter 1 bis 15 Gew.-% wirksame Substanz, bezogen auf das Gesamtgewicht der Zusammensetzung, verwendet wird.

16. Zusammensetzung nach einem der Ansprüche 1 bis 15, **dadurch gekennzeichnet, dass** der die Penetration fördernde Wirkstoff in Konzentrationen von 0,1 bis 25 Gew.-% und vorzugsweise 5 bis 10 Gew.-%, bezogen auf das Gesamtgewicht der Zusammensetzung, verwendet wird.

17. Zusammensetzung nach einem der Ansprüche 1 bis 16, **dadurch gekennzeichnet, dass** das Verhältnis der als wirksame Substanz ausgedrückten Mengenanteile der anionischen grenzflächenaktiven Stoffe und der amphoteren grenzflächenaktiven Stoffe im Bereich von 1 bis 20 und vorzugsweise 2 bis 10 liegt.

18. Zusammensetzung nach einem der Ansprüche 1 bis 17, **dadurch gekennzeichnet, dass** das Verhältnis der als wirksame Substanz ausgedrückten Mengenanteile der anionischen grenzflächenaktiven Stoffe und der die Penetration fördernden Wirkstoffe im Bereich von 0,1 bis 10 und vorzugsweise 0,5 bis 5 liegt.

19. Zusammensetzung nach einem der Ansprüche 1 bis 18, **dadurch gekennzeichnet, dass** die Zusammensetzung ferner kationische Polymere enthält, die unter den quaternisierten Proteinen, Polymeren vom Polyamintyp, Polyaminoamidtyp, quartären Polyammoniumtyp, Polyalkyleniminen, Kondensaten von Polyaminen und Epichlorhydrin, quartären Polyureylenen und Chitinderivaten ausgewählt sind.

20. Zusammensetzung nach Anspruch 19, **dadurch gekennzeichnet, dass** die kationischen Polymere unter den Derivaten von quartären Celluloseethern, Cyclopolymeren und kationischen Polysacchariden ausgewählt sind.

21. Zusammensetzung nach Anspruch 19 oder 20, **dadurch gekennzeichnet, dass** das kationische Polymer 0,001 bis 10 Gew.-%, vorzugsweise 0,005 bis 5 Gew.-% und noch bevorzugter 0,01 bis 3 Gew.-%, bezogen auf das Gesamtgewicht der Zusammensetzung, ausmacht.

22. Zusammensetzung nach einem der Ansprüche 1 bis 21, **dadurch gekennzeichnet, dass** der pH-Wert der Zusammensetzung im Bereich von 2 bis 9, vorzugsweise 3 bis 8 und noch bevorzugter 5,5 bis 6,5 liegt.

23. Zusammensetzung nach einem der Ansprüche 1 bis 22, **dadurch gekennzeichnet, dass** die Zusammensetzung auch nichtionische grenzflächenaktive Stoffe und/ oder Ceramide und/ oder Glykoceramide enthält.

24. Zusammensetzung nach einem der Ansprüche 1 bis 23, **dadurch gekennzeichnet, dass** die Zusammensetzung Verdickungsmittel enthält.

25. Zusammensetzung nach einem der Ansprüche 1 bis 24, **dadurch gekennzeichnet, dass** die Zusammensetzung auch Farbmittel, Viskositätsmodifizierer, Perlglanzstoffe, Hydratisierungsmittel, Wirkstoffe gegen Schuppen, Wirkstoffe gegen Seborrhoe, Sonnenschutzfilter, Proteine, Vitamine, α-Hydroxysäuren, Salze, Parfums, Konservierungsmittel, Maskierungsmittel, reizlindernde Stoffe, Schaummodifikatoren, klärende Stoffe oder deren Gemische enthält.

26. Zusammensetzung nach einem der Ansprüche 1 bis 25, **dadurch gekennzeichnet, dass** die Zusammensetzung auch Konditioniermittel enthält, wie hydrierte oder nicht hydrierte, synthetische oder nicht synthetische, auf Kohlenwasserstoffen basierende, zyklische oder aliphatische, geradkettige oder verzweigte (gesättigte oder ungesättigte) natürliche Öle, flüchtige oder nichtflüchtige, organomodifizierte oder nicht organomodifizierte, lösliche oder nicht lösliche Silicone, perfluorierte oder fluorierte Öle, Polybutene und Polyisobutene, Fettsäureester, die in flüssiger, pastöser oder fester Form vorliegen, Ester von mehrwertigen A1-koholen, Glyceride, natürliche oder synthetische Wachse, Silicongummis und Siliconharze, quartäre Salze oder Gemische dieser verschiedenen Stoffe.

27. Zusammensetzung nach Anspruch 26, **dadurch gekennzeichnet, dass** die Zusammensetzung ferner Quaternium-22 enthält.

28. Zusammensetzung nach einem der Ansprüche 1 bis 27, **dadurch gekennzeichnet, dass** sie in Form von Flüssigkeiten, die gegebenenfalls eingedickt sind, vorliegt.

29. Zusammensetzung nach einem der Ansprüche 1 bis 28 zur Anwendung als Arzneimittel.

30. Verwendung der Zusammensetzung nach einem der Ansprüche 1 bis 28 zur Herstellung eines Arzneimittels, das zur Behandlung von Erkrankungen der Kopfhaut vorgesehen ist.
